# EUROPEAN PATENT SPECIFICATION

(11) **EP 3 854 224 B1**
(45) Date of publication and mention of the grant of the patent: **17.12.2025**
(21) Application number: 18827732.1
(22) Date of filing: 21.09.2018
(51) Int. Cl.: A61K 35/741, A23L 11/00, A23L 17/60, A23L 19/00, A23L 19/10, A61K 31/15, A61K 31/19, A61K 36/03, A61K 36/062, A61K 36/07, A61K 36/16, A61K 36/185, A61K 36/28, A61K 36/282, A61K 36/288, A61K 36/29, A61K 36/31, A61K 36/39, A61P 35/00, A61P 43/00

(54) **IMMUNE CHECKPOINT SUPPRESSANT**
IMMUNCHECKPOINT-SUPPRESSOR
SUPPRESSEUR DE POINT DE CONTRÔLE IMMUNITAIRE

(43) Date of publication of application: 28.07.2021
(73) Proprietor: Nihon Sizen Hakkoh Co., Ltd., Takayama-shi, Gifu 501-5401 (JP)
(72) Inventor: HIGASHI, Naoki, Tokyo 145-0071 (JP); NAKANISHI, Masahiro, Takayama-shi Gifu 501-5401 (JP)
(74) Representative: Wächtershäuser & Hartz Patentanwaltspartnerschaft mbB
(86) International application number: PCT/JP2018/035179
(87) International publication number: WO 2019/009438

(56) References cited:
- WO-A1-02/072123
- WO-A1-2006/093267
- WO-A1-2016/093104
- WO-A1-2017/167213
- WO-A1-2017/167213
- WO-A2-2018/064165
- JP-A- 2002 138 046
- JP-A- 2004 107 286
- JP-A- 2006 117 575
- JP-A- 2007 084 504
- JP-A- 2007 230 973
- JP-A- 2007 319 157
- JP-A- 2008 255 057
- JP-A- 2013 237 652
- JP-A- 2015 202 065
- JP-A- H09 169 661
- US-A1- 2017 360 733

## Description

### Technical Field

The present invention relates to a plant fermentation product for use as an immune checkpoint suppressant.

### Background Art

Known cancer treatment methods include immunotherapy as well as surgical resection, anticancer agents mainly using various types of cytotoxin, hormone preparations, and radiotherapy.

Among the cancer treatment methods, the immunotherapy has conventionally been an auxiliary means. Strong candidates for the mechanism of immunotherapy include a mechanism relevant to the natural immunity by macrophage, or dendritic cells and a mechanism relevant to the acquired immunity in which killer T lymphocytes exhibit a killing effect on cancer cells and in which various T lymphocytes are involved. However, a mechanism in which killer T cells cannot sufficiently exhibit the effect has become apparent.

It has been actually reported that, when a ligand (PD-L1) which is produced by a cancer cell binds to a programed cell death-1 receptor (PD-1) on the surface of a suppressor T lymphocyte, the suppressor T cells proliferate to suppress the dysfunction effect of the killer T cells on cancer cells (NPL 1). This mechanism is referred to as immune checkpoint.

An immune checkpoint inhibitor which inhibits the immune checkpoint attracts attention as a new anticancer agent which is effective against various cancers that have conventionally been difficult to treat. Feature articles on the immune checkpoint inhibitor are published in technical journals of medical science (NPLs 2 to 3).

Many immune checkpoints have ever been discovered. Specific antibodies against such immune checkpoints have been approved one after another as a medicine, and have been broadly administered to cancer patients. However, the immune checkpoint inhibitors developed as a medicine and the substances under development are all specific antibodies. No substance other than specific antibodies has not been reported.

### Citation List

### Non-patent Literature

NPL 1: "Combination therapy strategies for improving PD-1 blockade efficacy: a new era in cancer immunotherapy" Chowdhury PS, Chamoto K, Honjo T, J. Interm Med, 2018 Feb; 283(2); 110-120. Doi: 10.1111/joim.12708.Equb.2017 Nov16.
NPL 2: "Gan wa Menekikei wo Ikani Yokusei Surunoka (how a cancer suppresses immune system", edited by NISHIKAWA Hiroyoshi, Jikken Igaku (experimental medical science), vol. 36(9), 2018, Yodosha
NPL 3: "Gan Menekiryoho - Syuyo Menekigaku no Saishinchiken kara Chiryoho no Appudeito made - (cancer immunotherapy - from the latest findings in tumor immunology to update of the therapy -", edited by KAWAKAMI Yutaka, Jikken Igaku Zokan (special issue of experimental medical science), vol. 34 (12), Yodosha 2016

WO 2017/167213 A1 discloses combined therapy of cancer using an immune checkpoint modulator and a fermented product prepared by symbiotic microbiota.

WO 2018/064165 A2 discloses compositions for the treatment of cancer by modulating the microbiome to enhance the efficacy of immune checkpoint blockade.

### Summary of Invention

### Technical Problem

Accordingly, the present invention which is defined by the claims, has an object to provide a novel immune checkpoint suppressant that has not been reported before.

### Solution to Problem

As a result of intensive and extensive studies for achieving the above object, the present inventors have found that a plant fermentation product (the present inventors have demonstrated that the plant fermentation product significantly achieves life extension and aging suppression and have already acquired a patent as an aging suppressant (Japanese Patent No. 6013670) ) acts as an immune checkpoint suppressant that has a different mechanism from that in conventional immune checkpoint inhibitors, completing the present invention.

The present invention relates to the subject matter of claims 1 to 10. Specifically, the present invention relates to a plant fermentation product for use as an immune checkpoint suppressant in a patient in need thereof containing, as an active ingredient a mixture of the following (a) to (g):
(a) a koji mold fermentation product of one or two or more beans or grains selected from the group consisting of barley, black soybean, red rice, black rice, adzuki bean, adlay, Japanese barnyard millet, foxtail millet, and proso millet;
(b) a yeast and/or lactic acid bacterium fermentation product of one or two or more fruits selected from the group consisting of mandarin orange, grape, apple, Vitis Coignetiae berry, peach, persimmon, papaya, Japanese pear, watermelon, Japanese apricot, fig, Chinese quince fruit, kabocha squash, kumquat, Yuzu, loquat fruit, apricot, jujube, chestnut, matatabi fruit, and Japanese plum;
(c) a yeast and/or lactic acid bacterium fermentation product of one or two or more root vegetables or potatoes selected from the group consisting of purple sweet potato, Jerusalem artichoke, carrot, onion, sweet potato, taro, Japanese wild yam, daikon, red turnip, great burdock, lotus root, yacon, lily bulb, arrowhead, ginger, garlic, and turmeric;
(d) a yeast and/or lactic acid bacterium fermentation product of one or two or more flowers or leafy vegetables selected from the group consisting of cabbage, perilla, Morus leaf, fish mint, Japanese mugwort, Sasa veitchii, and dandelion;
(e) a yeast and/or lactic acid bacterium fermentation product of one or two or more seaweeds selected from the group consisting of kelp, wakame, and mozuku;
(f) a yeast and/or lactic acid bacterium fermentation product of one or two or more seeds selected from the group consisting of black sesame, walnut, and ginkgo nut; and
(g) a yeast and/or lactic acid bacterium fermentation product of one or two or more mushrooms selected from the group consisting of maitake and shiitake.

The present invention also relates to the plant fermentation product for use as immune checkpoint suppressant further containing a therapeutic drug for a cancer and/or an alternative therapeutic drug for a cancer.

The present invention further relates to a food or drink containing the plant fermentation product, which food or drink is for use as an immune checkpoint suppressant in a patient in need thereof.

The present invention further relates to an agent for use in reducing an adverse reaction of radiotherapy, the agent containing the plant fermentation product for use as an immune checkpoint suppressant. Advantageous Effects of Invention

The present invention has obviously demonstrated that a certain plant fermentation product has a suppressing effect on an immune checkpoint. This is the first demonstration for a plant fermentation product.

The plant fermentation product of the present invention is for use in achieving treatment, prevention, or improvement of diseases associated with an immune checkpoint.

In addition, the plant fermentation product t of the present invention, which has lots of experiences as food, is suitable also for a food or drink, or a medicine.

### Brief Description of Drawings

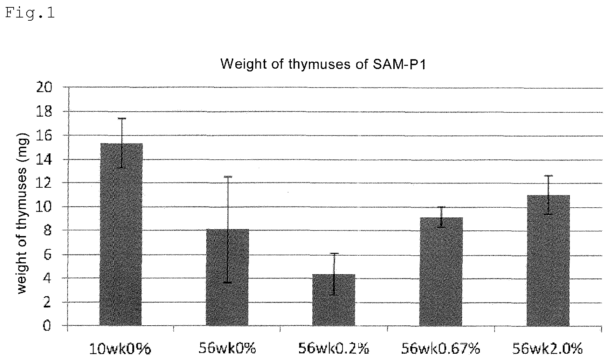

Fig. 1: It is a graph illustrating the average weight of thymuses in each group in Test Example 1.

### Description of Embodiments

The plant fermentation product for use as an immune checkpoint suppressant in a patient in need thereof is a mixture of the plant fermentation products of the following (a) to (g).

(a) A fermentation product obtained by allowing a koji mold to act on one or two or more beans or grains selected from the group consisting of barley, black soybean, red rice, black rice, adzuki bean, adlay, Japanese barnyard millet, foxtail millet, and proso millet.
(b) A fermentation product obtained by allowing a yeast and/or lactic acid bacterium to act on one or two or more fruits selected from the group consisting of mandarin orange, grape, apple, Vitis Coignetiae berry, peach, persimmon, papaya, Japanese pear, watermelon, Japanese apricot, fig, Chinese quince fruit, kabocha squash, kumquat, Yuzu, loquat fruit, apricot, jujube, chestnut, matatabi fruit, and Japanese plum.
(c) A fermentation product obtained by allowing a yeast and/or lactic acid bacterium to act on one or two or more root vegetables or potatoes selected from the group consisting of purple sweet potato, Jerusalem artichoke, carrot, onion, sweet potato, taro, Japanese wild yam, daikon, red turnip, great burdock, lotus root, yacon, lily bulb, arrowhead, ginger, garlic, and turmeric.
(d) A fermentation product obtained by allowing a yeast and/or lactic acid bacterium to act on one or two or more flowers or leafy vegetables selected from the group consisting of cabbage, perilla, Morus leaf, fish mint, Japanese mugwort, Sasa veitchii, and dandelion.
(e) A fermentation product obtained by allowing a yeast and/or lactic acid bacterium to act on one or two or more seaweeds selected from the group consisting of kelp, wakame, and mozuku.
(f) A fermentation product obtained by fermenting, with a yeast and/or lactic acid bacterium, one or two or more seeds selected from the group consisting of black sesame, walnut, and ginkgo nuts.
(g) A fermentation product obtained by allowing a yeast and/or lactic acid bacterium to act on one or two or more mushrooms selected from the group consisting of maitake and shiitake.

In the plant fermentation product of the present invention, in which the above (a) to (g) are mixed may be used as it is as an active ingredient, but the taste and the formulation capability can be enhanced by subjecting the plant fermentation product to further multistage fermentation.

Examples of the yeast include yeasts belonging to Saccharomyces, or Zygosaccharomyces. Among them, Saccharomyces cervisiae (S. cervisiae), Zygosaccharomyces rouxii (Z. rouxii), or Saccharomyces exiguus (S. exiguus are preferably used. Examples of the lactic acid bacterium include a lactic acid bacterium belonging to Pediococcus, Leuconostoc, or Lactobacillus, Lactococcus. Among them, Pediococcus acidilacti (P. acidilacti), Lactobacillus brevis (L. brevis), Leuconostoc mesenteroides (L. mesenteroides), Lactobacillus plantarum (L. plantarum), Lactococcus lactis (L. lactis), Lactobacillus sakei (L. sakei), Pediococcus pentosaceus (P. pentosaceus), Lactobacillus casei (L. casei), Lactobacillus reuteri (L. reuteri), and Lactobacillus curvatus (L. curvatus) are preferably used, and one or two or more thereof can be used. Examples of the koji mold include Aspergillus oryzae, Aspergillus niger, and Aspergillus kawachii, and one or two or more thereof can be used. A commercially available one can also be used.

A plant, such as a bean or grain or a fruit, to be subjected to the fermentation may be used as it is, or may be subjected to a pretreatment, such as pulverization or drying, as required. The plant may be diluted with water added, as required.

The fermentation products of the above (a) to (g) can be obtained by inoculating a lactic acid bacterium, yeast, or koji mold into a plant as a raw material, followed by culture. The culture may be conducted by an ordinary method, and, for example, a lactic acid bacterium, yeast, or koji mold is added in an amount of about 0.001 to 1% by mass to a mixture of one or two or more plants, which is then subjected to a fermentation treatment at 20 to 50°C for about 70 to 140 hours.

The thus obtained fermentation products of (a) to (g) are mixed, and the mixture may be used as it is as an active ingredient, but preferably the mixture is further cultured at 20 to 40°C for about 200 to 300 hours, as required. The mixture is preferably further subjected to post-fermentation (maturing). In the post-fermentation, an acetic bacterium may be allowed to act, as required. In an example, the aforementioned yeast is allowed to act on one or two or more of the plants included in the above (a) to (g), and an acetic bacterium, such as Acetobacter aceti (A. aceti), is allowed to act on the above product to obtain an acetic acid fermentation product, and the acetic acid fermentation product is added. The post-fermentation may be conducted at 25 to 35°C for 70 hours to about one year. Through the maturing process by the post-fermentation, the taste and the formulation capability can be improved and the antioxidative activity can also be increased.

A suitable example of a method for producing the plant fermentation product is a multistage composite fermentation system. In this system, a composite lactic acid bacterium fermentation product obtained by using a fruit, a vegetable, and/or a seaweed as a main raw material and a yeast fermentation product mainly obtained by using a vegetable, a root vegetable, a seed, a mushroom, and/or a fruit as a main raw material are mixed, and to the mixture, a koji mold fermentation product obtained by using a grain or bean as a main raw material is added, and an acetic acid fermentation product is further added, followed by mixing, and then the mixture was filtered and concentrated, and further subjected to post-fermentation for about one year. Through such multistage composite fermentation, each fermentation product is further assimilated and converted by different microorganisms, whereby the flavor is enhanced and the antioxidative activity and other effects are also enhanced.

The plant fermentation product as an active ingredient used in the present invention has properties of the following (1) to (4).

### (1) Taste

The plant fermentation product has sweetness due to a fruit, a vegetable, or another raw material and sweetness due to a koji mold as well as an organic acid. The plant fermentation product contains a polyphenol derived from a raw material but has less bitterness.

### (2) Solubility in water

The plant fermentation product is easily dissolved in water.

### (3) Stability

The plant fermentation product is stable against heat and acids and a paste thereof does not putrefy and is not changed in the taste even after preservation at room temperature for one year.

### (4) Safety

The vegetable, fruit, or herb used as a raw material, which are ordinary foods, and the yeast, lactic acid bacterium, and koji mold used in the fermentation, which are a microorganism derived from brewage of a food or from a pickle, have lots of experiences as food.

The plant fermentation product used in the present invention has properties of the following (i) to (iv).

### (i) Common components (based on 100 g)

| | |
|---|---|
| Water | 15 to 35 g |
| Protein | 5 to 20 g |
| Lipid | 1 to 8 g |
| Ash | 1 to 5 g |
| Carbohydrate | 30 to 70 g |
| Sodium | 40 to 150 mg |
| Vitamin B6 | 0.1 to 0.5 mg |
| Energy | 200 to 500 kcal |

### (ii) Amino acid composition (based on 100 g)

| | |
|---|---|
| Arginine | 0.2 to 0.6 g |
| Lysine | 0.1 to 0.7 g |
| Histidine | 0.1 to 0.4 g |
| Phenylalanine | 0.2 to 0.8 g |
| Tyrosine | 0.1 to 0.6 g |
| Leucine | 0.3 to 1.2 g |
| Isoleucine | 0.2 to 0.8 g |
| Methionine | 0.05 to 0.3 g |
| Valine | 0.2 to 0.9 g |
| Alanine | 0.2 to 0.9 g |
| Glycine | 0.2 to 0.7 g |
| Proline | 0.4 to 1.2 g |
| Glutamic acid | 1.2 to 3.0 g |
| Serine | 0.2 to 0.8 g |
| Threonine | 0.2 to 0.7 g |
| Aspartic acid | 0.4 to 1.5 g |
| Tryptophan | 0.03 to 0.15 g |
| Cystine | 0.05 to 0.40 g |

### (iii) Organic acid composition (based on 100 g)

| | | |
|---|---|---|
| | Citric acid | 0.5 to 1.2 g |
| | Malic acid | 0.05 to 0.5 g |
| | Succinic acid | 0.04 to 0.3 g |
| | Lactic acid | 0.5 to 6.0 g |
| | Formic acid | 0.01 to 0.1 g |
| | Pyruvic acid | 0.005 to 0.05 g |
| | Free γ-aminobutyric acid | 0.01 to 0.05 g |

### (iv) Mineral composition (based on 100 g)

| | |
|---|---|
| Phosphorus | 100 to 400 mg |
| Iron | 1 to 5 mg |
| Calcium | 500 to 900 mg |
| Potassium | 600 to 1000 mg |
| Magnesium | 70 to 120 mg |
| Zinc | 0.8 to 1.6 mg |
| Iodine | 1.0 to 2.5 mg |

The plant fermentation product as an active ingredient suppresses the expression of an immune checkpoint, for example, one or more selected from PD-1, PD-L1, and CTLA4, preferably all thereof, and more preferably PD-1 and PD-L1. Note that the plant fermentation product of the present invention, unlike existing immune checkpoint inhibitors, does not inhibit binding between a receptor and a ligand but suppresses the expression of the immune checkpoint, and furthermore, the immune checkpoint suppressant is of a new type that provides the effect by oral administration.

As described above, the plant fermentation product of the present invention, unlike existing immune checkpoint inhibitors, does not inhibit the binding between a receptor and a ligand, and by using a different mechanism that suppresses the expression of an immune checkpoint, the immune checkpoint suppressant can achieve treatment, prevention, or improvement of diseases associated with the immune checkpoint. Specifically, by suppressing the expression of PD-1 among immune checkpoints, the immune checkpoint suppressant can be used, for example, for treatment, prevention, or improvement of malignant melanoma, non-small-cell lung cancer, renal cell cancer, Hodgkin lymphoma, head and neck cancer, or gastric cancer, and for postoperative adjuvant therapy of malignant pleural mesothelioma or malignant melanoma. By suppressing the expression of CTLA4, the immune checkpoint suppressant can be used, for example, for treatment, prevention, or improvement of a melanoma, such as an untreated advanced melanoma, an unresectable melanoma, or metastasis melanoma, a non-small-cell lung cancer, or small cell lung cancer. By suppressing the expression of PD-L1, the plant fermentation product can be used, for example, for treatment, prevention, or improvement of unresectable advanced or recurrent non-small cell lung cancer. Note that the plant fermentation product of the present invention does not include a prophylactic agent for a spontaneous cancer for suppressing production of spontaneous cancers, which agents contain the same plant fermentation product as an active ingredient (an international patent has already been applied for the prophylactic agent for a spontaneous cancer (PCT/JP2018/ 34617)).

The plant fermentation product of the present invention is obtained by adding a pharmaceutically acceptable carrier, excipient, or water activity modifier to the thus-obtained plant fermentation product to make a formulation according to a known pharmaceutical production method. As required, the plant fermentation product may be concentrated to adjust the concentration, and may be made into powder by spray drying or freeze drying. Examples of the carrier or excipient used in the formulation include lactose, glucose, sucrose, starch, and a sugar mixture. Examples of the final form for use include solution, paste, soft capsule, chewable, and capsule. In an example of the dosage and usage, an adult orally takes about 0.1 g or more, preferably 0.1 to 12 g, more preferably 0.6 to 10 g (in terms of the solid) of the plant fermentation product as an active ingredient a day.

The plant fermentation product of the present invention can be made into the form of medicine, or quasi drug, or can be made into the form of food or drink by formulating a known food material therewith. The plant fermentation product can be taken as it is, but may be filtered and concentrated after sterilization for increasing the storability, or, as required, may be made into powder by adding an excipient, followed by spray drying or freeze drying. Furthermore, for increasing the shelf life during distribution, the plant fermentation product is preferably concentrated to reduce the water activity. Examples of the form of food or drink include paste, soft capsule, tablet, and drink. When an adult orally takes about 0.1 g or more, preferably 0.1 to 12 g, more preferably 0.6 to 10 g (in terms of solid) of the plant fermentation product a day, an excellent immune checkpoint suppressing effect can be achieved. Examples of commercially available products in which such a plant fermentation product is made into a formulation in the form of a food include Amo Koso (fermented botanical food) Kin-jirushi (gold label) and Amo Koso capsule (manufactured by Nihon Shizen Hakko Co. Ltd).

Besides the above products, the food or drink containing the plant fermentation product of the present invention may be a food or drink that contains the plant fermentation product which is an active ingredient as one of seasonings, examples of the food or drink including a seasoning, such as Miso; bread, such as pan loaf; sweets, such as rice crackers, cookies, chocolates, candies, sweet buns, and cakes; dairy products, such as yogurt and cheese; pickles, such as Takuan; noodles, such as Soba and Udon; soups, such as corn potage and Wakame soup; beverages, health drinks, carbonated drinks, and fruit juice drinks.

Furthermore, a therapeutic drug for a cancer and/or an alternative therapeutic drug for a cancer may further be incorporated into the plant fermentation product of the present invention to enhance the effect. The therapeutic drug for a cancer is not particularly limited, and examples thereof include a cytotoxic anticancer agent, such as an antimetabolite, an alkylation agent, an anticancer antibiotic, or a microtubule inhibitor, a molecular target therapeutic drug, a platinum-based drug, a topoisomerase inhibitor, and an immune checkpoint-specific inhibitor. The alternative therapeutic drug for a cancer is not particularly limited, and examples thereof include an herbal medicine, such as juzen-taiho-to, dai-saiko-to, or hochu-ekki-to, fucoidan, phellinus linteus, plant worm, Panax notoginseng, Deer horn shape ganoderma lucidum, Hedyotis diffusa, and fermentation agaricus. In particular, since the plant fermentation product of the present invention suppresses expression of immune checkpoints by a different mechanism from that of existing immune checkpoint inhibitors, the plant fermentation product is preferably used in combination with a therapeutic drug for a cancer having a strong adverse reaction of an autoimmunity increase, for example, ipilimumab which is a CTLA4-specific inhibitor. In addition, the plant fermentation product of the present invention is also preferably used in combination with a therapeutic drug for a cancer associated with strong immunotoxicity, for example, an alkylation agent, or with radiotherapy associated with strong immunotoxicity since the plant fermentation product as an active ingredient thereof has a suppressing action on the thymus atrophy. Thus, the adverse reactions of a therapeutic drug for a cancer and/or an alternative therapeutic drug for a cancer or radiotherapy can be reduced. When the therapeutic drug for a cancer and/or the alternative therapeutic drug for a cancer is incorporated in the plant fermentation product of the present invention, the content thereof may be appropriately set according to the intended effect.

### Examples

The present invention will be described in detail below with reference to examples, but the present invention is in no way limited to the examples.

### Production Example 1

### Production of plant fermentation product:

The following plants were used as raw materials.
(a) Bean or grain (barley, black soybean, red rice, black rice, adzuki bean, adlay, Japanese barnyard millet, foxtail millet, proso millet)
(b) Fruit (mandarin orange, grape, apple, Vitis Coignetiae berry, peach, persimmon, papaya, Japanese pear, watermelon, Japanese apricot, fig, Chinese quince fruit, kabocha squash, kumquats, Yuzu, loquat fruit, apricot, jujube, chestnut, matatabi fruit, Japanese plum)
(c) Root vegetable (purple sweet potato, Jerusalem artichoke, carrot, onion, sweet potato, taro, Japanese wild yam, daikon, red turnip, great burdock, lotus root, yacon, lily bulb, arrowhead, ginger, garlic, turmeric)
(d) Flower or leafy vegetable (cabbage, perilla, Morus leaf, fish mint, Japanese mugwort, Sasa veitchii, dandelion)
(e) Seaweed (kelp, wakame, mozuku)
(f) Seed (black sesame, walnut, ginkgo nut)
(g) Mushroom (maitake, shiitake)

Into raw materials of the above (c), (d), and (g) (730g), a culture fluid that was prepared so as to contain a lactic acid bacterium (P. acidilacti, L. brevis, L. mesenteroides, L. plantarum, L. lactis, L. sakei, L. casei) at a bacterial concentration of about 1.0 × 10⁵ cfu/g was inoculated at 0.2% by mass, followed by culture at 30°C for 50 hours. Meanwhile, into raw materials (900 kg) of the above (b), (e), and (f), a culture fluid that was prepared so as to contain a yeast (5 kinds of S. cervisiae, 2 kinds of Z. rouxii) at a bacterial concentration of about 1.0 × 10⁵ cfu/g was inoculated at 0.4% by mass, followed by culture at 30°C for 50 hours. Into a raw material (1000 kg) of (a) a bean or grain, a koji mold (yellow koji mold, black koji mold, white koji mold) was inoculated at 0.1% by mass, followed by culture at 35°C for 70 hours. Then, each culture was mixed, followed by culture at 30°C for 200 hours. The mash remaining after the fermentation was subjected to a solid-liquid separation operation, the resulting filtrate was concentrated into a paste, which was dispensed into containers, followed by post-fermentation (maturing) for further one year to obtain a plant fermentation product.

The plant fermentation product obtained in Production Example 1 had the following properties.

### (i) General analysis values (based on 100 g)

| | |
|---|---|
| Water | 25.2 g |
| Protein | 11.8 g |
| Lipid | 3.6 g |
| Ash | 2.1 g |
| Carbohydrate | 57.3 g |
| Sodium | 54.0 mg |
| Vitamin B6 | 0.20 mg |
| Energy | 309 kcal |

### (ii) Amino acid composition (based on 100 g)

A sample was hydrolyzed with 6 N hydrochloric acid, and was then analyzed with an amino acid automatic analyzer. For cystine, after a performic acid oxidation treatment, hydrochloric acid hydrolyzation was used. For tryptophan, high performance liquid chromatography was used.

| | |
|---|---|
| Arginine | 0.33 g |
| Lysine | 0.34 g |
| Histidine | 0.22 g |
| Phenylalanine | 0.51 g |
| Tyrosine | 0.32 g |
| Leucine | 0.74 g |
| Isoleucine | 0.42 g |
| Methionine | 0.13 g |
| Valine | 0.54 g |
| Alanine | 0.48 g |
| Glycine | 0.42 g |
| Proline | 0.92 g |
| Glutamic acid | 2.25 g |
| Serine | 0.4 g |
| Threonine | 0.36 g |
| Aspartic acid | 0.84 g |
| Tryptophan | 0.06 g |
| Cystine | 0.15 g |

### (iii) Organic acid composition (based on 100 g)

| | |
|---|---|
| Citric acid | 0.81 g |
| Malic acid | 0.31 g |
| Succinic acid | 0.12 g |
| Lactic acid | 1.17 g |
| Formic acid | 0.03 g |
| Pyruvic acid | 0.01 g |
| Free γ-aminobutyric acid | 24 mg |

### (iv) Mineral composition (based on 100 g)

| | |
|---|---|
| Phosphorus | 262 mg |
| Iron | 2.65 mg |
| Calcium | 72.1 mg |
| Potassium | 798 mg |
| Magnesium | 97.8 mg |
| Zinc | 1.19 mg |
| Iodine | 1.7 mg |

### Test Example 1

### Thymus atrophy suppressing test:

### (1) Mouse

Six SAMP1 (female) mice of 8 weeks old per group were purchased from Sankyo Labo Service Corporation and were used. The mice were raised in a mouse raising facility (23±2°C, humidity 50±10%, light-dark cycle of lighting from 20:00 to 8:00) according to an SPF specification. Six mice were used per group for each sample and were raised solely using a polycarbonate cage manufactured by CLEA Japan, Inc under SPF conditions. Feed was 500 N (sterilized with γ-ray) purchased from Sankyo Labo Service Corporation and was allowed to be freely taken. As a floor mat, a high-temperature-sterilized one manufactured by CLEA Japan, Inc. was used. The cage was replaced once a week. Note that the feed intake of the mice did not decrease in raising.

### (2) Sample

As a sample, a plant fermentation product in a paste form produced in Production Example 1 was used. The plant fermentation product was dissolved in ion exchange water at 2.0%, 0.2%, or 0% (control), and the solution was dispensed into 500-ml medium bottles, which were then all sterilized in an autoclave (121°C, 20 minutes). The supernatant was antiseptically transferred into a water supply bottle (sterilized) so that the nozzle of the water supply bottle was not clogged with generated precipitates, and was subjected to the test. Note that, with reference to a case in which a relatively larger amount of a plant fermentation product is routinely taken (77 g/week = 11 g/day), the amount of the plant fermentation product administered was determined by calculating a value corresponding to 30 times the above amount and converting the resulting value into an amount per Kg body weight. This conversion rate is a numerical value that is empirically used in Institute of Natural Medicine in University of Toyama when an herbal medicine is applied to mice. (Matsumoto Kinzo Personal Information, The 28th SAM workshop "Roka Moderu Doubutsu SAMP8 no Ninchi Kodo Syogai ni taisuru Kanpoyaku, Cho-to-san oyobi Cyuyaku Kangenkaryu no Kaizen Koka to Sorera no Sinkeikiko (improvement effect of herbal medicine, diao-teng-san and Chinese medicine, granular Kangen on cognition activity disorder of senescence model animal SAMP8 and neural mechanism thereof", July 5 (2013) , Aichi Gakuin University) . When a mouse drinks about 30 ml of a 2.0% concentration sample one day, the amount corresponds to about 10 g/day for a human. The plant fermentation product diluted with ion exchange water as described above was allowed to be freely taken as drinking water.

### (3) Anatomy

Young mice in the second week of raising (10 weeks old) and old mice in the 48th week of raising (56 weeks old) each were anesthetized with somnopentyl, and were then subjected to laparotomy to extract the thymus, the spleen, and the kidney which is not involved in the immunity, and the wet weight of each organ was measured. Fig. 1 shows an average weight of the thymuses in each group.

### (4) Result

Only the thymuses of the old mice had atrophy due to aging, and the weight was about one-half the weight of the thymuses of the young mice. The 0.2% plant fermentation product group had almost the same thymus weight as the 0% group, but in view of the result of the 2% group, the plant fermentation product suppressed the thymus atrophy in a concentration-dependent manner. However, the weight did not reach to the thymus weight of 10 weeks old. This demonstrated that the plant fermentation product can suppress atrophy of an immune organ due to aging.

### Test Example 2

### Flow cytometry analysis:

### (1) Mouse

Eight SAMP-1 (male) mice per group (1st) or nine mice per group (2nd) of 8 weeks old were purchased from Sankyo Labo Service Corporation and were used. The mice were raised solely in the same facility under the same conditions as in Test Example 1. Note that the feed intake of the mice did not decrease in raising.

### (2) Sample

The plant fermentation product in a paste form produced in Production Example 1 was used as a sample. The plant fermentation product was allowed to be freely taken in drinking water in the same manner as in Test Example 1 except that the plant fermentation product was dissolved in ion exchange water at a concentration of 2.0%, 0.4%, or 0% (control).

### (3) Flow cytometry

### <1st>

To young mice in the second week of raising (10 weeks old), a mixture of three types of anesthetic agents (medetomidine (0.3 mg/0.3 ml/Kg), midazolam (4 mg/0.8 ml/Kg), butorphanol (5 mg/1 ml/Kg) : final adjusted liquid amount 0.05 ml/10 g) was intraperitoneally administered, and then the mice were subjected to laparotomy to extract the whole blood from the descending artery, the thymus, and the spleen. The whole blood was refrigerated as it was and the thymus and the spleen were immersed in a preservation medium manufactured by Gibco (AIM-V medium: serum free), and the whole blood, thymus, and spleen were transported to a measurement facility (Kamakura Techno-Science, Inc., Department of Bio Research) with an ice bag.

A test was conducted for antibodies that broadly cover immune-related cells and a PD-1 antibody. As a flow cytometer, BD FACSCanto II was used. For the thymus and the spleen, RPMI1640 was added to disperse cells therein in a gentle MACS C tube with a gentle MACS dissociator. The cells were collected and then the number of WBCs was counted, and with HBSS, the concentration was adjusted to about 1 x 10⁷ cells/ml. The whole blood or the cell suspension 50 µl was dispensed into FACS tubes. An antibody mixture liquid was added and was incubated in a dark place at room temperature for 30 minutes. The resultant was subjected to an FACS analyzer, was developed by a CD45/SSC plotting, and was gated on CD45 positive cells. Subsequently, the WBCs were developed by FSC/SSC plotting and were gated on lymphocytes. All the samples were measured so that the taken cells were 10,000 lymphocytes. The analysis was performed using BD FACS Diva software Ver. **6.1.3.** and the analytical results were printed and taken as raw data. The ratio of the following cells in the whole blood, the thymus, and the spleen were calculated for each group. Table 1 shows the PD-1+T cell ratios (%) in the immune organ lymphocytes obtained by the flow cytometry.

### <Evaluated cell groups (macrophage and dendritic cells are omitted)>

T cells (CD3e)
B cells (CD19)
NK cell (CD49b)
Regulatory T cells (CD4+CD25+)
PD-1+CD44+CD4+T cells
Helper T cells (CD4+CD8-)
CTL T cells (CD4-CD8+)
DP T cells (CD4+CD8+)
DN T cells (CD4-CD8-)

### <Combination of antibodies to the above>

· CD45/CD3e/CD19/CD49b
· CD45/CD3e/CD4/CD8a/CD25/CD44/PD-1

**[Table 1]**

| Whole blood (peripheral blood) | | | | Thymus | | | | Spleen | | | |
|---|---|---|---|---|---|---|---|---|---|---|---|
| Sample 0% | | Sample 2% | | Sample 0% | | Sample 2% | | Sample 0% | | Sample 2% | |
| Normal | Tumor bearer | Normal | Tumor bearer | Normal | Tumor bearer | Normal | Tumor bearer | Normal | Tumor bearer | Normal | Tumor bearer |
| 8.1 | 16.3 | 13.1 | 22.0 | 1.7 | 16.3 | 1.6 | 4.0 | 19.9 | 31.5 | 12.5 | 19.6 |
| n=3 | n=3 | n=5 | n=1 | n=3 | n=3 | n=5 | n=1 | n=3 | n=3 | n=5 | n=1 |

In the water administration group (sample 0%), the PD-1+T cell ratios were higher in the tumor bearer in all the organs of the whole blood, the thymus, and the spleen. In the 2% administration (sample 2%), the PD-1+T cell ratios were low also in the tumor bearer except for one example. The one example was an osteosarcoma in a femoral region.

### <2nd>

A test was conducted for antibodies (CD45, CD3e, CD4, CD8a, CD25, FoxP3, and CD279 (PD-1)) with a focus on PD-1 and peripheral Treg. The cell groups were analyzed separately for the plant fermentation product concentrations of 0%, 0.4%, and 2.0%, and further separately for the tumor bearer and non-tumor bearer. Table 2 shows the PD-1+T cell ratios in the tumor bearers by the flow cytometry.

### <Evaluated cell groups>

1) Proportion of T cells in lymphocytes
2) Proportion of Treg cells (CD3+, CD4+, CD25+, Fox3+) in lymphocytes
3) Proportion of PD-1 positive Treg cells in lymphocytes
4) Proportion of CD4/PD-1 positive cells in lymphocytes
5) Proportion of helper T cells (CD3+, CD4+, CD8+) in T cells
6) Proportion of CTL (CD3+, CD4+, CD8+) in T cells
7) Proportion of PD-1 T cells (CD3+, CD279 (PD-1)+) in T cells

**[Table 2]**

| Mouse group | Immune organ | Plant fermentation product % | Normal body | Tumor bearer |
|---|---|---|---|---|
| 10 wk normal body (test start 8 weeks old) | Whole blood | 0 | 0.7 | - |
| | Thymus | 0 | 1.1 | - |
| | Spleen | 0 | 1.1 | - |
| 12SAM 71wk (test start = 14 weeks old) | Whole blood | 0 | 13.3 | 34.1 |
| | | 0.4 | 16.0 | 13.4 |
| | | 2.0 | 20.8 | 22.0 |
| | Thymus | 0 | 21.2 | 22.8 |
| | | 0.4 | 24.4 | 37.6 |
| | | 2.0 | 21.8 | 27.9 |
| | Spleen | 0 | 6.7 | 10.9 |
| | | 0.4 | 7.7 | 11.3 |
| | | 2.0 | 15.1 | 17.3 |

The PD-1 expression rates in the young (10 weeks old) mice were lower values (0.7 to 1.1) in all the whole blood, the thymus, and the spleen. In the 71 weeks old, the PD-1 expression rates were higher in all the whole blood, the thymus, and the spleen. The PD-1 expression rates in the spleen were about one-half those in the whole blood and the thymus. In the thymus, in all the sample concentration groups, the PD-1 expression rate values were higher in the tumor bearer, and the PD-1 expression rates decreased in a sample-concentration-dependent manner. The whole blood had a similar tendency, but the spleen had a different tendency from the thymus. The immune checkpoint expression increased with aging and was further higher in tumor bearers, but the expression was reduced in SAMP-1 mice which took the plant fermentation product.

### Test Example 3

### DNA Microarray (analysis of gene expression of immune checkpoint):

Six SAMP-1 (male) mice per group were raised to 10 weeks old or raised for a long period of time to 56 weeks old while giving 0% (ion exchange water) or 2.0% of the plant fermentation product in drinking water, and were anesthetized with somnopentyl, followed by laparotomy to extract the thymus and the kidney. The thymus and the kidney were immersed in an ice-cooled RNA later solution and were transported to a measurement facility (Filgen, Inc.) in a refrigerated state. Operations from the RNA extraction through the DNA microarray measurement were conducted in the measurement facility.

Among the analysis data obtained from the above facility, the data for 7 molecules of sirtuin which was said to be related to extension of life span were checked. For all of the combinations of 10 weeks old or 56 weeks old, the thymus or the kidney, and the plant fermentation product 0% or 2%, no increase or decrease was recognized in the sirtuin gene expression and thus it was determined that sirtuin does not relate to the life extension effect.

In terms of the degree of the increase or decrease in the gene expression and in the frequency thereof, there were variations in various regions of molecules of immunoglobulin, but the characteristics of the antibodies were not able to be detected. However, it was apparent that the immunity was related. Variations in the small nuclear RNA were also notable. Although there is no cited document, a relation to the signaling in the nuclear is presumed to be involved in the life extension phenomenon. This was however not able to be analyzed in detail. It was found from the results that the plant fermentation product does not cause any variation in a conventionally known gene, such as sirtuin.

### Test Example 4

### Analyses of immune checkpoint by DNA microarray:

The results of the flow cytometry revealed the relation of PD-1 which is an immune checkpoint. Thus, a DNA microarray probe that relates to an immune checkpoint was tried to be extracted from DNA microarray probes. As a result, three types were included. Specifically, Ctla4 (corresponding to CTLA-4), Pdcd1 (corresponding to PD-L1), and Cd274 (corresponding to PD-1) were included in the database. The relevant data obtained in Test Example 3 was subjected to reanalysis from a different viewpoint. Thus, for the 10 weeks old versus the 56 weeks old in the thymus and the kidney, and for the 0% concentration versus the 2.0% concentration of the plant fermentation product, the gene expressions of the three genes were compared. An increase or decrease was seen only in the thymus, and there was no change at all in the kidney. Table 3 shows the results on the thymus.

**[Table 3]**

| Database of DNA microarray | Abbreviation of immune checkpoint | 0%-10 wk vs 0%-56 wk | 0%-10 wk vs 2%-56 wk | 0%-56 wk vs 2%-56 wk |
|---|---|---|---|---|
| Ctla4 | corresponding to CTLA-4 | 1.4844 | 1.4262 | 0.9608 |
| Pdcd1 | corresponding to PD-L1 | 1.1805 | 1.0735 | 0.9094 |
| Cd274 | corresponding to PD-1 | 1.5240 | 1.3702 | 0.8991 |

It is interesting that PD-L1 which is a ligand simultaneously exhibited a similar behavior to PD-1 which is a receptor. Both the specific antibodies to the PD-1 and PD-L1 have been approved as a medicine as an immune checkpoint inhibitor. As compared with young mice (10 weeks old), the old mice showed about 1.3-fold increase in the log of expressions of all the three types of genes. The increase was suppressed into a decrease (about 0.9 in the log) in the 2% group as compared to the 0% group, and in particular, the tendency was obvious in PD-1 and PD-L1. Ctla4 showed the same tendency but the degree was smaller than in the PD-1 system. In the kidney, no increase or decrease was observed for the three genes, and a different behavior was shown from the thymus which is the center of the immunoregulation.

In addition, in the 56 weeks old, the expressions of the three types of immune checkpoints were increased both in 0% or 2%. The ratio of PD-1 and PD-L1 (Cd274) tended to be lower in the 2% administration group, but the ratio of CTLA-4 was changed less. In the 56 weeks old, the immune checkpoints were lower in the 2% administration group than in the 0% group. In the kidney, no increase or decrease was observed in the expression of the three types of genes, and the increase or decrease was specific to the thymus. That is, it was found that two receptors and a ligand involved in an immune checkpoint of the thymus lymphocyte are increased with aging. The increment was lower in the mice which took 2% of the plant fermentation product. Furthermore, between the old mice (56 weeks old), the expression was obviously lower in the 2% plant fermentation product taking group. The rates of increase or decrease of PD-1 and PD-L1 were clear as compared with CTLA4. It was found from the results that, while the gene expressions of PD-1, PD-L1, and Ctla4 in the thymus generally increase with aging, the increments are all reduced by the plant fermentation product.

### Test Example 5

### Inhibitory activity on binding of PD-1 receptor and ligand (PD-L1 and PD-L2):

A binding test was conducted in vitro using a binding assay kit manufactured by BPS Bioscience, Inc. which can achieve a binding test of two ligands, PD-L1 and PD-L2, to a mouse-derived PD-1 receptor. The detection was performed using Alpha LISA manufactured by Perkin Elmer Co., Ltd. Since the paste of the plant fermentation product produced in Production Example 1 used in the test had a brown color, the color developing method with peroxidase was avoided. A 10% aqueous solution of the plant fermentation product was subjected to autoclave sterilization and the supernatant was collected by an Eppendorf centrifuge. Note that, since this sample contained a miner amount of biotin, the sample was mixed with strptoavidin mag sepharose washed with PBS to remove the biotin in the sample. The resulting aqueous solution was defined as a 10% sample solution. This solution was serially diluted with miliQ water and the dilutions were subjected to a test. The color development was measured using Optiplate-384 manufactured by Perkin Elmer, Co., Ltd. The binding inhibition activity was checked using the PD-1-specific antibody as a positive control.

The PD-1-specific antibody actually exhibited concentration-dependent direct inhibition, which showed that the test was capable of achieving a normal evaluation. The IC50% in this test was about 2.5 nM. On the other hand, the plant fermentation product exhibited no inhibitory activity. The plant fermentation product exhibits a quite different behavior from conventionally known immune checkpoint inhibitors and exhibits such an attitude to the binding reaction of the immune checkpoint. In addition to that, no substance that suppresses the PD-1 expression in vivo has been known.

### Example 1

### Miso-like food:

The plant fermentation product in a paste form produced in Production Example 1 was mixed with Miso and the taste was adjusted with seasonings to produce a Miso-like food.

### Example 2

### Drink:

The plant fermentation product in a paste form produced in Production Example 1 was dissolved in water, which was then mixed with a fructose glucose liquid and a fruit juice, and the taste was adjusted to produce a drink.

### Example 3

### Soft capsule:

The plant fermentation product in a paste form produced in Production Example 1 was mixed with a vegetable oil and lecithin, which was filled in a soft capsule by an ordinary method to produce a soft capsule.

### Example 4

### Chewable tablet:

The plant fermentation product in a paste form produced in Production Example 1 was mixed with crystalline cellulose and white sugar, which was tableted by an ordinary method to produce a chewable tablet.

### Industrial Applicability

According to the present invention, an immune checkpoint can be suppressed. Thus, the present invention is useful for treatment, prevention, or improvement of diseases associated with the immune checkpoint.

## Claims

1. A plant fermentation product for use as an immune checkpoint suppressant in a patient in need thereof, wherein the plant fermentation product comprises, as an active ingredient, a mixture of (a) to (g):
(a) a koji mold fermentation product of one or two or more beans or grains selected from the group consisting of barley, black soybean, red rice, black rice, adzuki bean, adlay, Japanese barnyard millet, foxtail millet, and proso millet;
(b) a yeast and/or lactic acid bacterium fermentation product of one or two or more fruits selected from the group consisting of mandarin orange, grape, apple, Vitis Coignetiae berry, peach, persimmon, papaya, Japanese pear, watermelon, Japanese apricot, fig, Chinese quince fruit, kabocha squash, kumquat, Yuzu, loquat fruit, apricot, jujube, chestnut, matatabi fruit, and Japanese plum;
(c) a yeast and/or lactic acid bacterium fermentation product of one or two or more root vegetables or potatoes selected from the group consisting of purple sweet potato, Jerusalem artichoke, carrot, onion, sweet potato, taro, Japanese wild yam, daikon, red turnip, great burdock, lotus root, yacon, lily bulb, arrowhead, ginger, garlic, and turmeric;
(d) a yeast and/or lactic acid bacterium fermentation product of one or two or more flowers or leafy vegetables selected from the group consisting of cabbage, perilla, Morus leaf, fish mint, Japanese mugwort, Sasa veitchii, and dandelion;
(e) a yeast and/or lactic acid bacterium fermentation product of one or two or more seaweeds selected from the group consisting of kelp, wakame, and mozuku;
(f) a yeast and/or lactic acid bacterium fermentation product of one or two or more seeds selected from the group consisting of black sesame, walnut, and ginkgo nut; and
(g) a yeast and/or lactic acid bacterium fermentation product of one or two or more mushrooms selected from the group consisting of maitake and shiitake.

2. The plant fermentation product for use according to claim 1, wherein the plant fermentation product comprises amino acids in the following composition based on 100 g of the plant fermentation product:
| | |
|---|---|
| arginine | 0.2 to 0.6 g |
| lysine | 0.1 to 0.7 g |
| histidine | 0.1 to 0.4 g |
| phenylalanine | 0.2 to 0.8 g |
| tyrosine | 0.1 to 0.6 g |
| leucine | 0.3 to 1.2 g |
| isoleucine | 0.2 to 0.8 g |
| methionine | 0.05 to 0.30 g |
| valine | 0.2 to 0.9 g |
| alanine | 0.2 to 0.9 g |
| glycine | 0.2 to 0.7 g |
| proline | 0.4 to 1.2 g |
| glutamic acid | 1.2 to 3.0 g |
| serine | 0.2 to 0.8 g |
| threonine | 0.2 to 0.7 g |
| aspartic acid | 0.4 to 1.5 g |
| tryptophan | 0.03 to 0.15 g |
| cystine | 0.05 to 0.40 g. |

3. The plant fermentation product for use according to claim 1 or 2, wherein the plant fermentation product comprises organic acids in the following composition based on 100 g of the plant fermentation product:
| | |
|---|---|
| citric acid | 0.5 to 1.2 g |
| malic acid | 0.05 to 0.5 g |
| succinic acid | 0.04 to 0.3 g |
| lactic acid | 0.5 to 6.0 g |
| formic acid | 0.01 to 0.1 g |
| pyruvic acid | 0.005 to 0.05 g |
| free γ-aminobutyric acid | 0.01 to 0.05 g. |

4. The plant fermentation product for use according to any one of claims 1 to 3, wherein the lactic acid bacterium is one or two or more selected from the group consisting of Pediococcus acidilacti (P. acidilacti), Lactobacillus brevis (L. brevis), Leuconostoc mesenteroides (L. mesenteroides), Lactobacillus plantarum (L. plantarum), Lactococcus lactis (L. lactis), Lactobacillus sakei (L. sakei), Pediococcus pentosaceus (P. pentosaceus), Lactobacillus casei (L. casei), and Lactobacillus curvatus (L. curvatus).

5. The plant fermentation product for use according to any one of claims 1 to 4, wherein the yeast is Saccharomyces cervisiae (S. cervisiae) and/or Zygosaccharomyces rouxii (Z. rouxii).

6. The plant fermentation product for use according to any one of claims 1 to 5, wherein the immune checkpoint suppressant suppresses expression of one or more selected from PD-1, PD-L1, and CTLA4.

7. The plant fermentation product for use according to any one of claims 1 to 6, further comprising a therapeutic drug for a cancer and/or an alternative therapeutic drug for a cancer.

8. A food or drink comprising the plant fermentation product according to any of claims 1 to 7, for use as an immune checkpoint suppressant in a patient in need thereof.

9. The plant fermentation product for use according to any one of claim 1 to 7, wherein the plant fermentation product is a medicine.

10. An agent for use in reducing an adverse reaction of radiotherapy, the agent comprising the plant fermentation product for use according to any one of claims 1 to 7.

## Patentansprüche

1. Pflanzliches Fermentationsprodukt zur Verwendung als Immun-Checkpoint-Suppressor bei einem Patienten, der dies benötigt, wobei das pflanzliche Fermentationsprodukt als Wirkstoff eine Mischung aus (a) bis (g) umfasst:
(a) ein Koji-Schimmel-Fermentationsprodukt aus einer oder zwei oder mehr Bohnen oder Körnern, ausgewählt aus der Gruppe bestehend aus Gerste, schwarzer Sojabohne, rotem Reis, schwarzem Reis, Adzuki-Bohne, Adlay, japanischer Hühnerhirse, Fuchsschwanzhirse und Prosohirse;
(b) ein Hefe- und/oder Milchsäurebakterien-Fermentationsprodukt aus einer oder zwei oder mehreren Früchten, ausgewählt aus der Gruppe bestehend aus Mandarine, Traube, Apfel, Vitis Coignetiae-Beere, Pfirsich, Persimone, Papaya, japanischer Birne, Wassermelone, japanischer Aprikose, Feige, chinesischer Quitte, Kabocha-Kürbis, Kumquat, Yuzu, Loquat-Frucht, Aprikose, Jujube, Kastanie, Matatabi-Frucht und japanischer Pflaume;
(c) ein Hefe- und/oder Milchsäurebakterien-Fermentationsprodukt aus einem oder zwei oder mehreren Wurzelgemüsen oder Kartoffeln, ausgewählt aus der Gruppe bestehend aus violetter Süßkartoffel, Topinambur, Karotte, Zwiebel, Süßkartoffel, Taro, japanischer Wildyam, Daikon, roter Rübe, großer Klette, Lotuswurzel, Yacon, Lilienknolle, Pfeilspitze, Ingwer, Knoblauch und Kurkuma;
(d) ein Hefe- und/oder Milchsäurebakterien-Fermentationsprodukt aus einer oder zwei oder mehreren Blumen oder Blattgemüsen, ausgewählt aus der Gruppe bestehend aus Kohl, Perilla, Maulbeerbaumblatt, Fischminze, japanischem Beifuß, Sasa veitchii und Löwenzahn;
(e) ein Hefe- und/oder Milchsäurebakterien-Fermentationsprodukt aus einer oder zwei oder mehreren Algen, ausgewählt aus der Gruppe bestehend aus Seetang, Wakame und Mozuku;
(f) ein Hefe- und/oder Milchsäurebakterien-Fermentationsprodukt aus einem oder zwei oder mehreren Samen, ausgewählt aus der Gruppe bestehend aus schwarzem Sesam, Walnuss und Ginkgo-Nuss; und
(g) ein Hefe- und/oder Milchsäurebakterien-Fermentationsprodukt aus einem oder zwei oder mehreren Pilzen, ausgewählt aus der Gruppe bestehend aus Maitake und Shiitake.

2. Das pflanzliche Fermentationsprodukt zur Verwendung gemäß Anspruch 1, wobei das pflanzliche Fermentationsprodukt Aminosäuren in der folgenden Zusammensetzung, bezogen auf 100 g des pflanzlichen Fermentationsprodukts, umfasst:
| | |
|---|---|
| Arginin | 0,2 bis 0,6 g |
| Lysin | 0,1 bis 0,7 g |
| Histidin | 0,1 bis 0,4 g |
| Phenylalanin | 0,2 bis 0,8 g |
| Tyrosin | 0,1 bis 0,6 g |
| Leucin | 0,3 bis 1,2 g |
| Isoleucin | 0,2 bis 0,8 g |
| Methionin | 0,05 bis 0,30 g |
| Valin | 0,2 bis 0,9 g |
| Alanin | 0,2 bis 0,9 g |
| Glycin | 0,2 bis 0,7 g |
| Prolin | 0,4 bis 1,2 g |
| Glutaminsäure | 1,2 bis 3,0 g |
| Serin | 0,2 bis 0,8 g |
| Threonin | 0,2 bis 0,7 g |
| Asparaginsäure | 0,4 bis 1,5 g |
| Tryptophan | 0,03 bis 0,15 g |
| Cystin | 0,05 bis 0,40 g |

3. Das pflanzliche Fermentationsprodukt zur Verwendung gemäß Anspruch 1 oder 2, wobei das pflanzliche Fermentationsprodukt die folgenden organischen Säuren in der folgenden Zusammensetzung, bezogen auf 100 g des pflanzlichen Fermentationsprodukts, umfasst:
| | |
|---|---|
| Zitronensäure | 0,5 bis 1,2 g |
| Apfelsäure | 0,05 bis 0,5 g |
| Bernsteinsäure | 0,04 bis 0,3 g |
| Milchsäure | 0,5 bis 6,0 g |
| Ameisensäure | 0,01 bis 0,1 g |
| Brenztraubensäure | 0,005 bis 0,05 g |
| freie γ-Aminobuttersäure | 0,01 bis 0,05 g. |

4. Das pflanzliche Fermentationsprodukt zur Verwendung gemäß einem der Ansprüche 1 bis 3, wobei das Milchsäurebakterium eines oder zwei oder mehrere aus der Gruppe ausgewählt ist, die aus Pediococcus acidilacti (P. acidilacti), Lactobacillus brevis (L. brevis), Leuconostoc mesenteroides (L. mesenteroides), Lactobacillus plantarum (L. plantarum), Lactococcus lactis (L. lactis), Lactobacillus sakei (L. sakei), Pediococcus pentosaceus (P. pentosaceus), Lactobacillus casei (L. casei) und Lactobacillus curvatus (L. curvatus) ausgewählt sind.

5. Das pflanzliche Fermentationsprodukt zur Verwendung gemäß einem der Ansprüche 1 bis 4, wobei die Hefe Saccharomyces cervisiae (S. cervisiae) und/oder Zygosaccharomyces rouxii (Z. rouxii) ist.

6. Das pflanzliche Fermentationsprodukt zur Verwendung gemäß einem der Ansprüche 1 bis 5, wobei der Immun-Checkpoint-Suppressor die Expression von einem oder mehreren ausgewählten aus PD-1, PD-L1 und CTLA4 unterdrückt.

7. Das pflanzliche Fermentationsprodukt zur Verwendung gemäß einem der Ansprüche 1 bis 6, das ferner ein Therapeutikum für Krebs und/oder ein alternatives Therapeutikum für Krebs umfasst.

8. Lebensmittel oder Getränk, das das pflanzliche Fermentationsprodukt zur Verwendung gemäß einem der Ansprüche 1 bis 7 umfasst zur Verwendung als Immun-Checkpoint-Suppressor in einem Patienten, der dies benötigt.

9. Das pflanzliche Fermentationsprodukt zur Verwendung gemäß einem der Ansprüche 1 bis 7, wobei das pflanzliche Fermentationsprodukt ein Arzneimittel ist.

10. Mittel zur Verwendung bei der Verringerung einer Nebenwirkung der Strahlentherapie, wobei das Mittel das pflanzliche Fermentationsprodukt zur Verwendung gemäß einem der Ansprüche 1 bis 7 umfasst.

## Revendications

1. Produit de fermentation végétale à utiliser comme suppresseur de point de contrôle immunitaire chez un patient qui en a besoin, dans lequel le produit de fermentation végétale comprend, en tant qu'ingrédient actif, un mélange de (a) à (g) :
(a) un produit de fermentation de moisissure de koji composé d'un ou deux haricots ou grains ou plus choisis dans le groupe consistant en l'orge, le soja noir, le riz rouge, le riz noir, le haricot adzuki, l'adlay, le millet japonais, le millet à queue de renard, et le millet commun ;
(b) un produit de fermentation de levure et/ou de bactérie lactique d'un ou deux fruits ou plus choisis dans le groupe consistant en la mandarine, le raisin, la pomme, la baie de Vitis Coignetiae, la pêche, le kaki, la papaye, la poire japonaise, le melon d'eau, l'abricot japonais, la figue, le coing chinois, la courge kabocha, le kumquat, le yuzu, la bibasse, l'abricot, le jujube, la châtaigne, le fruit de matatabi et la prune japonaise ;
(c) un produit de fermentation de levure et/ou de bactérie lactique d'un ou deux légumes racines ou pommes de terre ou plus choisis dans le groupe consistant en la patate douce violette, le topinambour, la carotte, l'oignon, la patate douce, le taro, l'igname sauvage japonaise, le daikon, le navet rouge, la grande bardane, la racine de lotus, la poire de terre, la bulbe de lys, la sagittaire, le gingembre, l'ail et le curcuma ;
(d) un produit de fermentation de levure et/ou de bactérie lactique d'un(e) ou deux fleurs ou légumes à feuilles ou plus choisis dans le groupe consistant en le chou, la périlla, la feuille de murier, la menthe de poisson, l'armoise japonaise, le Sasa veitchii et le pissenlit ;
(e) un produit de fermentation de levure et/ou de bactérie lactique d'une ou deux algues marines ou plus choisies dans le groupe consistant en le varech, le wakame et le mozuku ;
(f) un produit de fermentation de levure et/ou de bactérie lactique d'une ou deux graines ou plus choisies dans le groupe consistant en le sésame noir, la noix et la noix de ginkgo ; et
(g) un produit de fermentation de levure et/ou de bactérie lactique d'un ou deux champignons ou plus choisis dans le groupe consistant en le maitake et le shiitake.

2. Produit de fermentation végétale à utiliser selon la revendication 1, dans lequel le produit de fermentation végétale comprend des acides aminés dans la composition suivante sur la base de 100 g du produit de fermentation végétale :
| | |
|---|---|
| arginine | 0,2 à 0,6 g |
| lysine | 0,1 à 0,7 g |
| histidine | 0,1 à 0,4 g |
| phénylalanine | 0,2 à 0,8 g |
| tyrosine | 0,1 à 0,6 g |
| leucine | 0,3 à 1,2 g |
| isoleucine | 0,2 à 0,8 g |
| méthionine | 0,05 à 0,30 g |
| valine | 0,2 à 0,9 g |
| alanine | 0,2 à 0,9 g |
| glycine | 0,2 à 0,7 g |
| proline | 0,4 à 1,2 g |
| acide glutamique | 1,2 à 3,0 g |
| sérine | 0,2 à 0,8 g |
| thréonine | 0,2 à 0,7 g |
| acide aspartique | 0,4 à 1,5 g |
| tryptophane | 0,03 à 0,15 g |
| cystine | 0,05 à 0,40 g. |

3. Produit de fermentation végétale à utiliser selon la revendication 1 ou 2, dans lequel le produit de fermentation végétale comprend des acides organiques dans la composition suivante sur la base de 100 g du produit de fermentation végétale :
| | |
|---|---|
| acide citrique | 0,5 à 1,2 g |
| acide malique | 0,05 à 0,5 g |
| acide succinique | 0,04 à 0,3 g |
| acide lactique | 0,5 à 6,0 g |
| acide formique | 0,01 à 0,1 g |
| acide pyruvique | 0,005 à 0,05 g |
| acide γ-aminobutyrique libre | 0,01 à 0,05 g. |

4. Produit de fermentation végétale à utiliser selon l'une quelconque des revendications 1 à 3, dans lequel la bactérie lactique est une ou deux ou plus choisies dans le groupe consistant en Pediococcus acidilacti (P. acidilacti), Lactobacillus brevis (L. brevis), Leuconostoc mesenteroides (L. mesenteroides), Lactobacillus plantarum (L. plantarum), Lactococcus lactis (L. lactis), Lactobacillus sakei (L. sakei), Pediococcus pentosaceus (P. pentosaceus), Lactobacillus casei (L. casei) et Lactobacillus curvatus (L. curvatus).

5. Produit de fermentation végétale à utiliser selon l'une quelconque des revendications 1 à 4, dans lequel la levure est Saccharomyces cervisiae (S. cervisiae) et/ou Zygosaccharomyces rouxii (Z. rouxii).

6. Produit de fermentation végétale à utiliser selon l'une quelconque des revendications 1 à 5, dans lequel le suppresseur de point de contrôle immunitaire supprime l'expression d'un ou plusieurs parmi PD-1, PD-L1 et CTLA4.

7. Produit de fermentation végétale à utiliser selon l'une quelconque des revendications 1 à 6, comprenant en outre un médicament thérapeutique pour un cancer et/ou un médicament thérapeutique alternatif pour un cancer.

8. Aliment ou boisson comprenant le produit de fermentation végétale selon l'une quelconque des revendications 1 à 7, à utiliser comme suppresseur de point de contrôle immunitaire chez un patient qui en a besoin.

9. Produit de fermentation végétale à utiliser selon l'une quelconque des revendications 1 à 7, dans lequel le produit de fermentation végétale est un médicament.

10. Agent à utiliser pour réduire une réaction indésirable de radiothérapie, l'agent comprenant le produit de fermentation végétale à utiliser selon l'une quelconque des revendications 1 à 7.
